# EUROPEAN PATENT SPECIFICATION

(11) **EP 2 840 981 B1**
(45) Date of publication and mention of the grant of the patent: **10.04.2019**
(21) Application number: 13709654.1
(22) Date of filing: 27.02.2013
(51) Int. Cl.: A61B 17/32, A61M 1/00

(54) **SURGICAL INSTRUMENT**
CHIRURGISCHES INSTRUMENT
INSTRUMENT CHIRURGICAL

(30) Priority: 26.04.2012 US 201213457006
(43) Date of publication of application: 04.03.2015
(73) Proprietor: Gyrus ACMI, Inc., Bartlett, TN 38133 (US)
(72) Inventor: EDWARDS, Kevin C., Olive Branch, MS 38654 (US); BENNETT, Michael J., Bartlett, TN 38135 (US); TEO, Chee K., Memphis, TN 38135 (US); FLYNN, John P., Collierville, TN 38017 (US); PALMER, Allen C., Arlington, TN 38002 (US); REIF, Matthias, 22455 Hamburg (DE); RYAN, Phil A., Memphis, TN 38117 (US)
(74) Representative: Wallin, Nicholas James
(86) International application number: PCT/US2013/028003
(87) International publication number: WO 2013/162699

(56) References cited:
- WO-A1-95/21641
- US-A1- 2005 197 645
- US-A1- 2006 264 995

## Description

### BACKGROUND

This invention relates to surgical instruments that remove matter such as cut biological tissue from a surgical site by suction. In particular, this invention relates to surgical instruments that, in addition to providing suction, include a positive pressure providing source to remove an obstruction such as cut biological material from the surgical instruments during a surgical procedure.

Surgical instruments used to shave, cut, resect, abrade and/or remove tissue, bone and or other bodily materials are known. Such surgical instruments can include a cutting surface, such as a rotating/reciprocating blade, disposed on an elongated inner tube that is rotated/reciprocated within an elongated outer tube having a cutting window. The inner and outer tubes together forming a surgical cutting blade. In general, the elongated outer tube includes a distal end defining an opening or cutting window that exposes the cutting surface of the inner tube (at the distal end of the inner tube) to tissue, bone and/or any other bodily materials.

Once the tissue, bone and/or any other bodily materials are cut, it is necessary to remove them from the surgical site. This is typically accomplished through an inner lumen provided in the surgical instrument that is connected to a suction source. Existing surgical instruments utilize a one-way suction line to aspirate tissue from the surgical site. It is often the case that the tissue forms a clog in the aspiration pathway (most often in the distal end of the surgical instrument). One way to remove the clog is for the person using the surgical instrument to remove the instrument from the surgical site and insert a stylet into the cutting window to force the clog through the cutting portion and into the inner lumen to be aspirated. However, this is often a time-consuming and tedious process, particularly over the course of a surgical procedure in which the surgeon may have to remove multiple clogs. Furthermore, removing and reinserting the surgical instrument is traumatic to the surgical site.

U.S. Patent Application Publication No. 2006/0264995 A1 discloses various embodiments in which, when a clog is detected in a surgical cutting instrument, flow in aspiration tubing is interrupted and the aspiration tubing is compressed such that a positive pressure is provided to unclog the surgical cutting instrument (see Figs. 2A-8). U.S. Patent Application Publication No. 2006/0264995 A1 also discloses embodiments in which a valve cuts off suction to the surgical cutting instrument and then a separate actuation of a means for flushing causes pressurized fluid to be provided to unclog the surgical cutting instrument (see Figs. 18A-21B). However, the various embodiments disclosed in U.S. Patent Application Publication No. 2006/0264995 A1 can be either difficult to use in practice or are inadequate to fully clear a clog quickly and efficiently.

U.S. Patent Application Publication No. 2005/0197645 A1 discloses an apparatus for vacuum -assisted irrigation and drainage of a body cavity including a poppet valve. The apparatus provides irrigation and aspiration through a single line and serves to reduce problems associated with clogging of the aspiration line or the inlet to said line.

Therefore, in view of the above-mentioned problems associated with clearing clogs from a surgical instrument, it is desirable to develop ways of clearing clogs quickly and efficiently.

### SUMMARY

In view of the above, it is desirable to provide a surgical instrument having a mechanism to remove a clog using a fluid (air or liquid) to create a back pressure that will blow out the debris and clear a pathway in the surgical instrument. Preferred embodiments are capable of removing clogs by one action of the user (e.g., by actuating a pressurization mechanism that results in providing pressure and changing a pathway at one time) and through a relatively simple structure.

The present invention provides a surgical instrument as defined in appended claim 1.

Preferred embodiments are defined in the appended dependent claims.

The surgical instrument includes a hollow tubular member having a cutting blade disposed on a distal end thereof. A handpiece is connected to a proximal end of the hollow tubular member and has a suction passage that connects to the hollow tubular member. A suction pump is configured to aspirate tissue that is cut by the cutting blade. A pressurization unit is provided between the suction passage and the suction pump. The pressurization unit includes an outer valve portion having a first port that connects to the suction passage and a second port that connects to the suction pump, and a pressurization device that is connected to the outer valve portion. The pressurization device has a movable part that is configured to generate pressurized fluid when the movable part is moved. An inner valve portion is slidably provided within the outer valve portion and is configured to move from a first position to a second position. The inner valve portion includes a first flow passage configured to connect the first port and the second port of the outer valve portion to each other when the inner valve portion is in the first position, and a second flow passage configured to connect the first port of the outer valve portion to the pressurization device when the inner valve portion is in the second position. The pressurization unit is configured such that the pressurized fluid from the pressurization device causes the inner valve portion to move from the first position to the second position, thereby providing positive pressure to the hollow tubular member.

The inner valve portion may be biased towards the first position by a spring that provides a biasing force to the inner valve portion. The pressurization device may be a compressed air cylinder, a manually compressible syringe or a manually compressible inflation bulb.

The present invention provides a method for cleaning an obstruction from a surgical instrument as defined in appended claim 9. Preferred embodiments are defined in the appended dependent claims.

A method for clearing an obstruction from a surgical instrument includes providing a hollow tubular member having a cutting blade disposed on a distal end thereof, and providing a handpiece connected to a proximal end of the hollow tubular member and having a suction passage that connects to the hollow tubular member. The method also includes providing a suction pump that is configured to aspirate tissue that is cut by the cutting blade, and providing a pressurization unit between the suction passage and the suction pump. The pressurization unit includes an outer valve portion having a first port that connects to the suction passage and a second port that connects to the suction pump, and a pressurization device connected to the outer valve portion. The pressurization device has a movable part configured to generate pressurized fluid when the movable part is moved, and an inner valve portion slidably provided within the outer valve portion and configured to move from a first position to a second position. The inner valve portion includes a first flow passage configured to connect the first port and the second port of the outer valve portion to each other when the inner valve portion is in the first position; and a second flow passage that is configured to connect the first port of the outer valve portion to the pressurization device when the inner valve portion is in the second position. The method further includes clearing an obstruction from the surgical instrument by actuating the movable part, which causes the pressurized fluid from the pressurization device to move the inner valve portion from the first position to the second position thereby providing positive pressure to the hollow tubular member.

### BRIEF DESCRIPTION OF THE DRAWINGS

Various exemplary embodiments of the disclosed surgical instrument will be described in detail with reference to the following drawings in which:
Fig. 1 illustrates a perspective view of a powered surgical tool system that incorporates a controller, a power source, a suction source, a pressurization unit and a surgical instrument having a handpiece;
Fig. 2 illustrates a distal end portion of the surgical instrument including a cutting portion;
Fig. 3 illustrates a pressurization unit in a suction position according to a first embodiment according to the invention;
Fig. 4 illustrates the pressurization unit according to the first embodiment while in a de-clog position;
Fig. 5 illustrates a pressurization unit in a suction position according to a second embodiment according to the invention;
Fig. 6 illustrates the pressurization unit according to the second embodiment while in a de-clog position;
Fig. 7 illustrates a pressurization unit in a suction position according to a third embodiment according to the invention;
Fig. 8 illustrates a flow chart of a de-clog process;
Fig. 9 illustrates a pressurization unit in a suction position according to an exemplary fourth embodiment, not in accordance to the invention;
Fig. 10 illustrates the pressurization unit according to the exemplary fourth embodiment while in a de-clog position;
Fig. 11 illustrates a pressurization unit in a suction position according to an exemplary fifth embodiment, not in accordance to the invention;
Fig. 12 illustrates the pressurization unit according to the exemplary fifth embodiment while in a de-clog position;
Fig. 13 illustrates an exemplary embodiment, not in accordance to the invention, having a switch unit other than a trumpet valve;
Fig. 14 illustrates another exemplary embodiment, not in accordance to the invention, having a switch unit other than a trumpet valve;
Figs. 15a-15d illustrate various types of exemplary switches including a rocker switch and a sliding switch;
Figs. 16a-16b illustrate an exemplary embodiment, not in accordance to the invention, where a rotary switch switches between providing suction and providing a positive pressure;
Fig. 17 illustrates a pressurization unit according to an exemplary sixth embodiment, not in accordance to the invention;
Fig. 18 illustrates the pressurization unit according to the exemplary sixth embodiment while in a suction providing state; and
Fig. 19 illustrates the pressurization unit according to the exemplary sixth embodiment while in a positive pressure providing (de-clog) state.

### DETAILED DESCRIPTION OF EMBODIMENTS

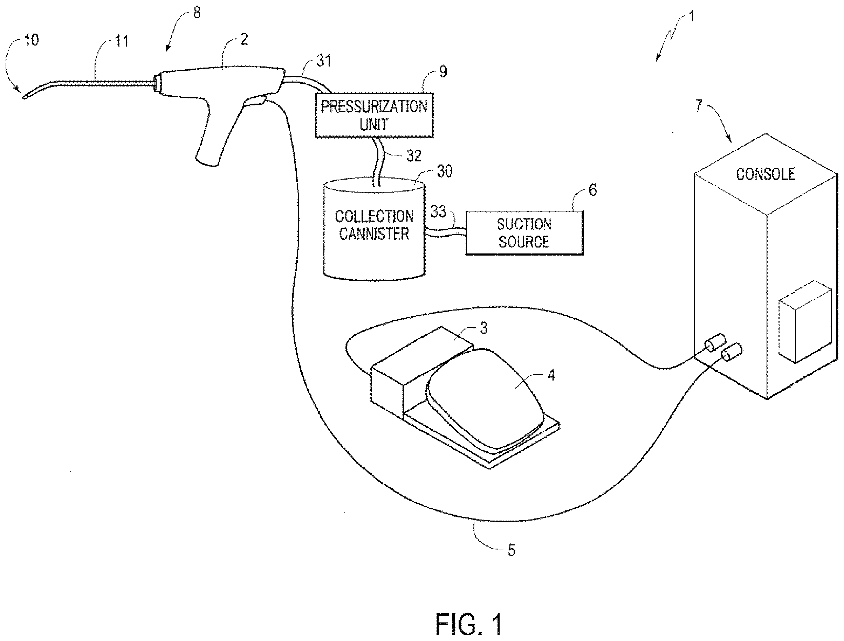

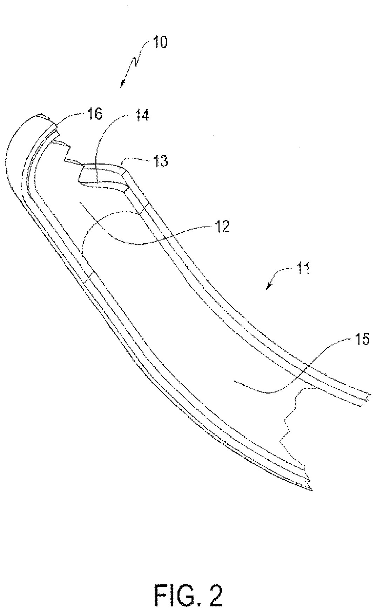

Fig. 1 is a schematic of a powered surgical tool system 1. The powered surgical tool system 1 includes a footswitch 3 (with pedal 4), a suction source 6, a console 7, a pressurization unit 9 and a surgical instrument 8 having a handpiece 2. The console 7 contains a power supply for the handpiece 2. A handpiece power cable 5 between the handpiece 2 and the console 7 is the electrical connection that powers a drive motor inside the handpiece 2. A power cord from a wall outlet plugs into the console 7 to power it. The suction source 6 may be a suction pump or any other suction providing source. The handpiece 2 is connected, at its distal end, to a surgical tool 10, which includes a hollow tubular member 11. The surgical tool 10 may be a microdebrider that includes a cutting blade 12 (see Fig. 2) at its distal end that is used to cut, shave, remove, resect and/or abrade tissue, bone and/or other bodily materials. As seen in Fig. 1, the pressurization unit 9 may be provided between the suction source 6 and the handpiece 2.

A collection canister 30 is provided between the suction source 6 and the pressurization unit 9. The pressurization unit 9 is connected to the handpiece 2 by a first suction tube 31, the collection canister 30 is connected to the pressurization unit 9 by a second suction tube 32, and the collection canister 30 is connected to the suction source 6 by a third suction tube 33. Preferably the second suction tube 32 is longer than the first suction tube 31. For example, the first suction tube 31 may be approximately 18 inches such that the pressurization unit 9 is in the sterile field for actuation by the surgeon and/or a surgical assistant. On the other hand, the second suction tube 32 may be approximately 10 feet in length such that the end of the suction tubing can easily reach the collection canister 30.

Fig. 2 illustrates a cross-sectional view of a distal end of the surgical tool 10 in which an inner tube 12 is co-axially disposed within an outer tube 13. The inner tube 12 includes a suction passage 15 that extends the length of the inner tube 12. The inner tube 12 also includes a cutting blade 14 while the outer tube 13 includes a cutting window 16 at a distal end. The inner tube 12 is co-axially disposed within the outer tube 13 such that the cutting blade 14 is exposed at the cutting window 16. The cutting blade 14 disposed within the cutting window 16 form a cutting portion, which cuts by rotating/reciprocating the inner tube 12 within the outer tube 13. Suction is provided through the suction passage 15 to remove tissue, bone and/or any other bodily materials from the surgical site and to convey the removed material into the collection canister 30. Alternatively, pressurized fluid can be provided through the suction passage 15 to clear, if necessary, a clog in the suction passage 15 or at a distal end of the surgical tool 10.

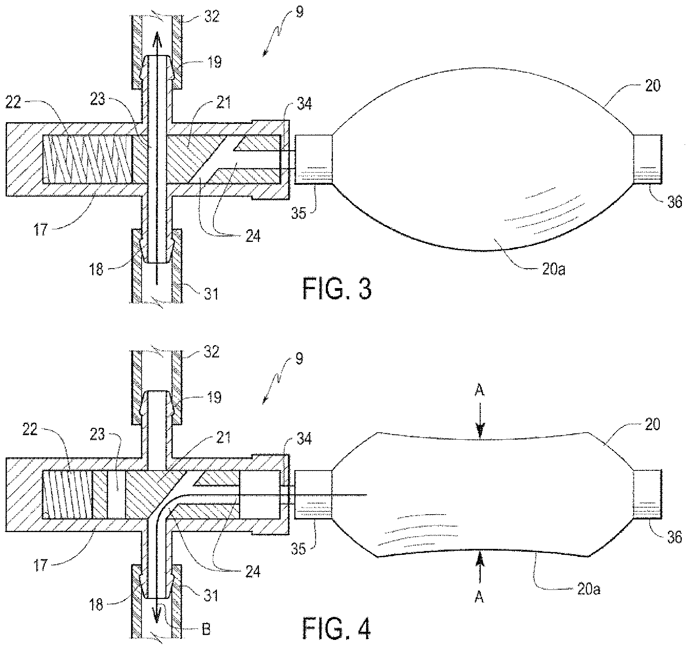

Fig. 3 illustrates the pressurization unit 9 according to a first embodiment of the invention. In the embodiment illustrated in Fig. 3, the pressurization unit 9 includes an outer valve portion 17 that has a first port 18 that connects to the handpiece 2, and ultimately to the suction passage 15 of the inner tube 12, and a second port 19 that connects to the suction source 6 via the collection canister 30. The pressurization unit 9 also includes a pressurization device 20 that is connected to a third port 34 of the outer valve portion 17. In the embodiment illustrated in Fig. 3, the pressurization device 20 is a manually compressible inflation bulb. The pressurization device 20 has a movable control part 20a, which is the compressible part of the manually compressible inflation bulb.

An inner valve portion 21 is slidably provided within the outer valve portion 17 so as to be able to move from a first position to a second position. The inner valve portion 21 is biased towards the first position (illustrated in Fig. 3) preferably by means of a spring 22. However, the inner valve portion 21 may be biased towards the first position by a rubber gasket instead of a spring or by any other suitable biasing means. The inner valve portion 21 includes a first flow passage 23 and a second flow passage 24.

When the inner valve portion 21 is in the first position (illustrated in Fig. 3), suction is provided to the hollow tubular member 11 via the first port 18, the first flow passage 23 and the second port 19, which connects to the suction source 6 via the collection canister 30.

As seen in Fig. 4, when the movable part 20a of the pressurization device 20 is compressed in the direction of arrows A, positive pressure emitted from the inflation bulb into a passage at the third port 34 (the right end of the outer valve portion 17 in Figs. 3 and 4) causes the inner valve portion 21 to slide in a direction toward pressure flow (leftward in Figs. 3 and 4) within the outer valve portion 17 against the biasing force of the spring 22 to move the inner valve portion 21 from the first position to the second position. This causes the positive pressure emitted from the inflation bulb to pass in the direction of arrow B into the second flow passage 24, out the first port 18 and to the hollow tubular member 11, thereby causing a clog to be cleared from the hollow tubular member 11. The clog may also be cleared from any other portion of the surgical tool 10 between the pressurization unit 9 and the cutting window 16 (e.g, in the first suction tube 31). The positive pressure provided by compressing the pressurization device 20a can be any type of fluid such as either a gas or a liquid. The inflation bulb preferably includes a first one-way valve 35 (e.g., a flapper valve) at its exit aperture (on the left side in Figs. 3 and 4) so that when the inflation bulb is squeezed, the pressurized fluid moves out of the inflation bulb and into the outer valve portion 17. The inflation bulb also preferably has a second one-way valve 36 (e.g., a flapper valve) at its entrance aperture (on the right side of the inflation bulb in Figs. 3 and 4) so that when the bulb is released, fresh fluid enters (and fills) the bulb from the right side. Accordingly, fluid from the surgical site will not enter the inflation bulb, protecting the inflation bulb from contamination.

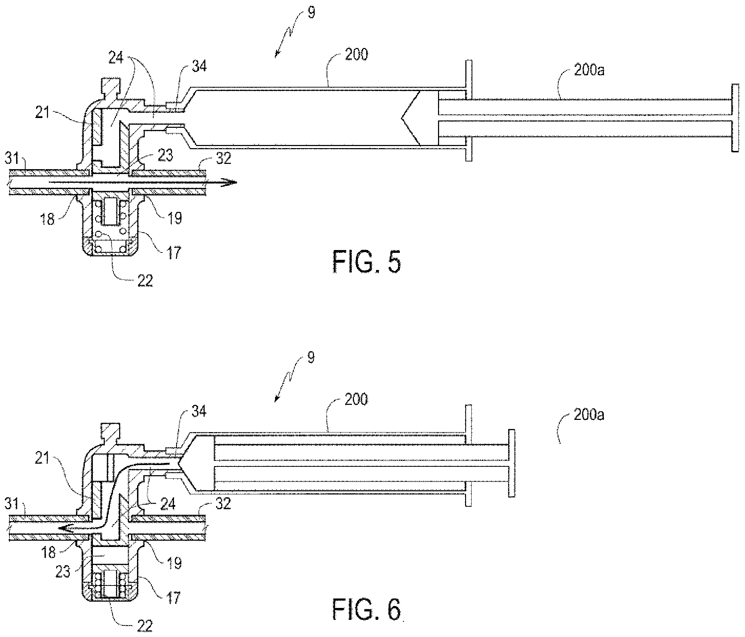

Fig. 5 illustrates a second embodiment of the invention, which is similar to the first embodiment except that the pressurization device 20 is a syringe 200 instead of a manually compressible inflation bulb. The syringe 200 includes a movable part (plunger) 200a that, when depressed in the direction of arrow C (see Fig. 6), causes the inner valve portion 21 to move from the first position to the second position and, as a result, causes positive pressure to be provided to clear a clog. Similar to the embodiment illustrated in Fig. 3, preferably a spring 22 biases the inner valve portion 21 towards the first position. However, negative pressure created by retracting the plunger 200a could be used to move the inner valve portion 21 towards the first position. The syringe 200 may also be configured to be removed and filled with fluid prior to being reattached to the outer valve portion 17.

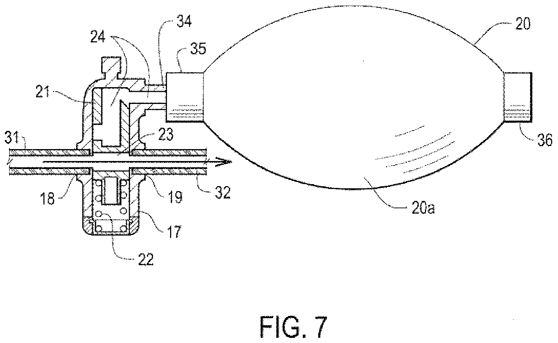

Fig. 7 illustrates a third embodiment of the invention in which the outer valve portion 17 and inner valve portion 21 are similar to that illustrated in Figs. 5 and 6, but an inflation bulb is provided as the pressurization device 20 instead of a syringe. The inflation bulb functions in the same manner as described with respect to the first embodiment.

Instead of using an inflation bulb or a syringe, a compressed air cylinder could be used as the pressurization device. Rotating the compressed air cylinder (for example by 90 degrees) would cause pressurized gas to be emitted from the cylinder into the outer valve portion 17 to cause the inner valve portion 21 to move from the first (suction) position to the second (de-clog) position. Rotating the cylinder back to its original position would stop the flow of pressurized gas and return the inner valve portion 21 to the suction position. A stopcock, push-button, or other manually actuated 2-position valve could be incorporated between the air cylinder and the pressurization device to control flow of gas from the air cylinder.

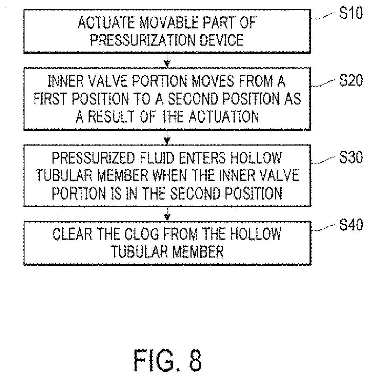

Fig. 8 is a flow chart illustrating a process in which a clog may be cleared from, for example, the hollow tubular member 11, with reference being had to the first embodiment of the invention. In step S10, the movable part 20a of the pressurization device 20 is actuated (compressed). In step S20, as a result of the actuation of the movable part 20a of the pressurization device 20, the inner valve portion 21 automatically slides from the first position to the second position, thus changing the locations of the first flow passage 23 and the second flow passage 24. In step S30, pressurized fluid is provided to the hollow tubular member 11 as a result of the actuation of the movable part 20a. In step S40, the clog is cleared as a result of the actuation of the movable part 20a causing the inner valve portion 21 to move and causing the supply of pressurized fluid into the suction passage of the surgical instrument. The suction from the suction source 6 does not need to be stopped during the de-clog process. Instead, the suction is merely blocked briefly until the inner valve portion 21 moves back from the second position to the first position. Accordingly, the disclosed system is easy to construct and use.

The embodiments described above are advantageous because they are very easy for the surgeon to use. In particular, the surgeon needs to perform a single operation (squeeze the inflation bulb, press the plunger, or turn (or similarly actuate, depending on the flow control mechanism) the compressed air cylinder) in order to change the flow path (suction or pressure) attached to the surgical instrument's suction passage and to supply the pressure. The inflation bulb embodiment is especially convenient because it automatically resets itself when the surgeon releases the bulb. Clogs are removed without having to withdraw the surgical instrument from the surgical site. Further, the inflation bulb embodiments are advantageous because inflation bulbs are intuitive to use, ergonomic and can be operated with a single hand.

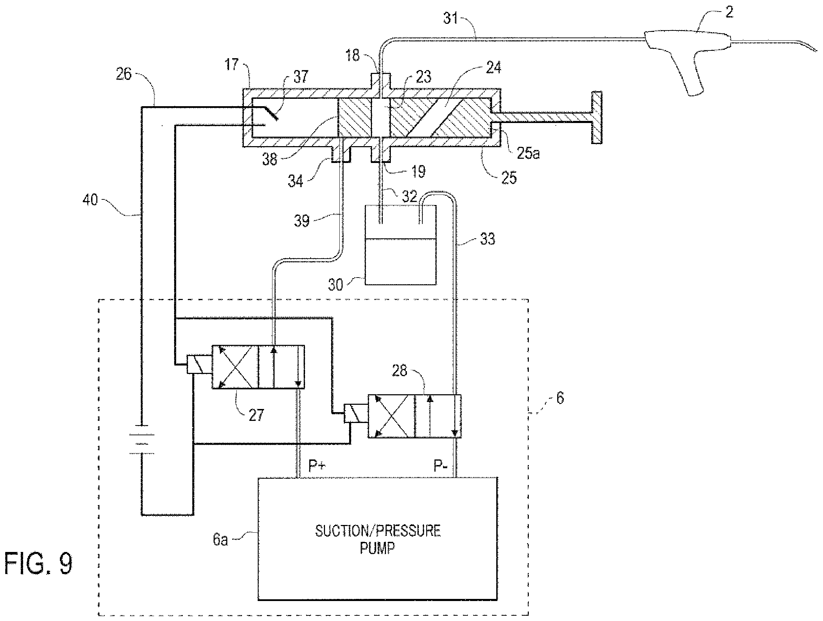

Fig. 9 illustrates an exemplary fourth embodiment, not in accordance with the invention, having a switching unit 25 that is configured to switch between a suction providing state and a positive pressure providing state. The switching unit 25 illustrated in Fig. 9 is similar to the pressurization device 20 of the first embodiment except that a contact head 38 of a movable part 25a of the switching unit 25 switches a switch 37 to complete a circuit 26 when the movable part 25a is moved toward the switch 37 (to the left-most in Fig. 9), de-clog position, to cause a suction/pressure pump 6a of the suction source 6 to switch from providing suction to providing positive pressure. This is accomplished by selectively energizing and de-energizing solenoid valves 27 and 28. Fig. 9 illustrates a de-energized position in which suction is provided from the suction/pressure pump 6a through the switching unit 25 and to the hollow tubular member 11 of the handpiece 2. Thus, according to the exemplary fourth embodiment, a single movement of the movable part 25a causes the suction source 6 to switch from providing suction to providing positive pressure.

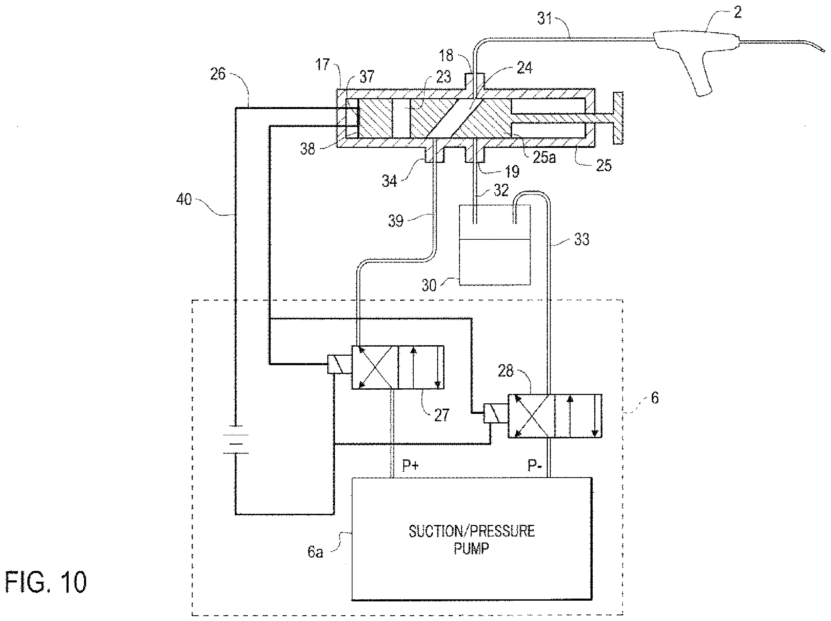

In addition to the second suction tube 32, the exemplary fourth embodiment includes a pressure tube 39 and an electrical communication cord 40 that are attached to the pressurization unit 9 (the switching unit 25 and the pump). A pump 6a may be used as part of the suction source 6. For many common pumps, there is a +P and a -P outlet as the pump mechanism brings in ambient air through the -P side and expels the air through the +P side. The exemplary fourth embodiment takes advantage of both sides of the pump, whereas alternative embodiments may only connect to the P side. When the switch 37 is an open position (see Fig. 9), the solenoid valve 27 is closed such that no air passes through the solenoid valve 27 whereas solenoid valve 28 is open to allow suction through solenoid valve 28. When the switch 37 is closed (see Fig. 10), the solenoid valves 27 and 28 are moved so as to allow pressurized air to flow through solenoid valve 27 whereas no suction flows through solenoid valve 28. The solenoid valves 27 and 28 and the pump 6a may be provided in a portable housing, which can include its own power source or may include a plug for obtaining power from an electrical outlet. Suction tubing from the collection canister 30, the third suction tube 33, the pressure tube 39 and the electrical communication cord plug 40 may enter into the pump housing of the suction source 6. As an alternative to the single pump 6a illustrated in Figs. 9 and 10, two pumps, one using only the +P side and the other using only the -P side, may be provided. This prevents cross contamination of air from past patients that may be contained inside a singular pump and recirculated to a patient in a subsequent surgery during a positive pressurization de-clog cycle.

Fig. 10 illustrates the exemplary fourth embodiment in an energized position in which the movable part 25a is depressed causing the first flow passage 23 to be switched to the second flow passage 24 while at the same time causing the contact head 38 to close the switch 37 and causing the completion of the circuit 26. This causes activation of the solenoid valves 27 and 28, which results in positive pressure being provided from a positive pressure providing port of the suction/pressure pump 6a through the pressure tube 39 ,the second flow passage 24 of switching unit 25 and the first suction tube 31 to the handpiece 2 to clear a clog.

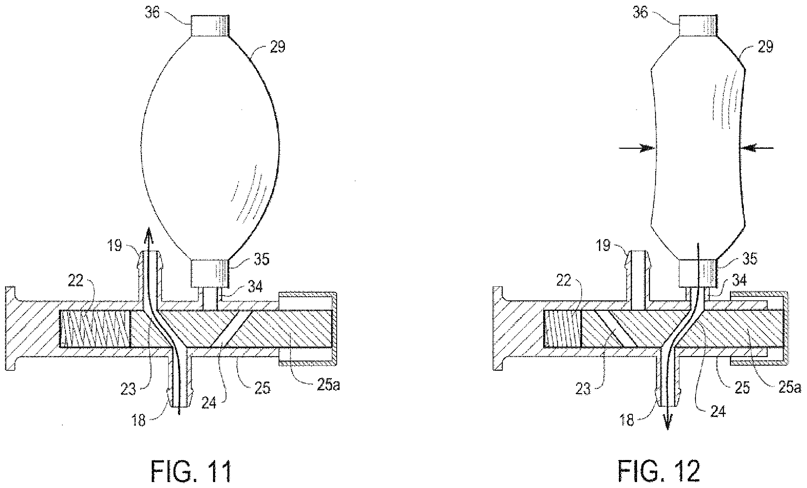

Figs. 11 and 12 illustrate an exemplary fifth embodiment, not in accordance with the invention, having a switching unit 25 that is similar to that illustrated in the exemplary fourth embodiment except that, instead of providing positive pressure via a positive pressure providing port of the suction/pressure pump 6a, a manually compressible inflation bulb 29 is provided. After the movable part 25a is depressed in Fig. 12, pressurized fluid can be provided through the second flow passage 24 to the handpiece 2 by compressing the manually compressible inflation bulb 29. A syringe or compressed air cylinder could alternatively be used in the exemplary embodiment illustrated in Figs. 11 and 12 instead of the manually compressible inflation bulb 29. The exemplary fourth and fifth embodiments are different from the first, second and third embodiments in that, in the exemplary fourth and fifth embodiments, the movement of the inner valve portion 21 of the switch unit 25 is not caused by the positive fluid pressure.

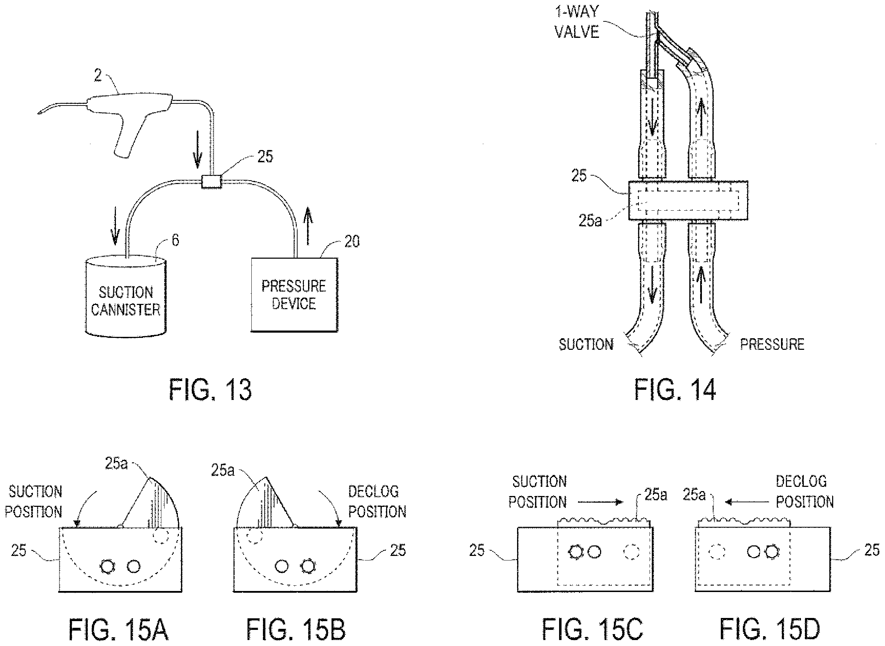

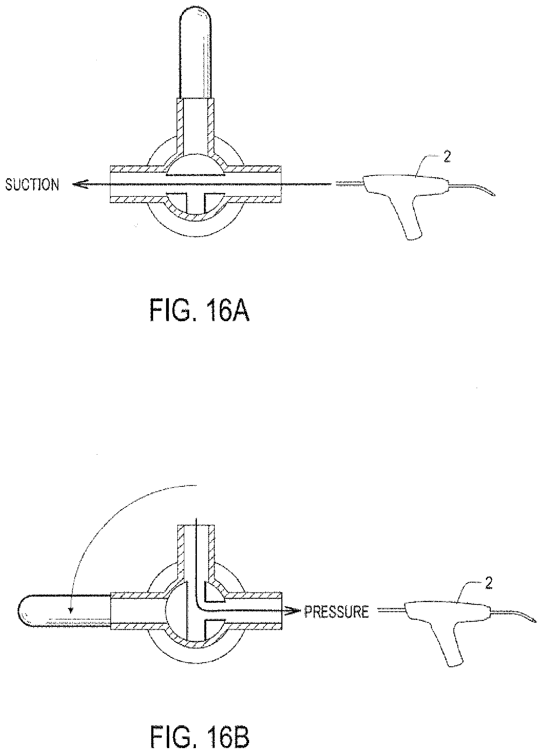

The exemplary fourth and fifth embodiments illustrated in Figs. 9-12 utilize a trumpet style valve for the switching unit 25 to switch between providing suction and positive pressure. Instead of a trumpet style valve, various other types of switching devices may be used for the switching unit 25. For example, Figs. 13 and 14 illustrate a switching unit 25 that is different than a trumpet valve. In particular, the switching unit 25 may be a rotary switch, a rocker switch or a sliding switch. Figs. 15a-15d illustrate a rocker switch and a sliding switch. Fig. 15a illustrates a suction providing state of the rocker switch. Fig. 15b illustrates a de-clog providing state of the rocker switch. Figs. 15c-15d illustrate a slider switch that can slide to selectively provide suction or allow positive pressure therethrough. Fig. 16a and 16b illustrate different states of a rotary switch.

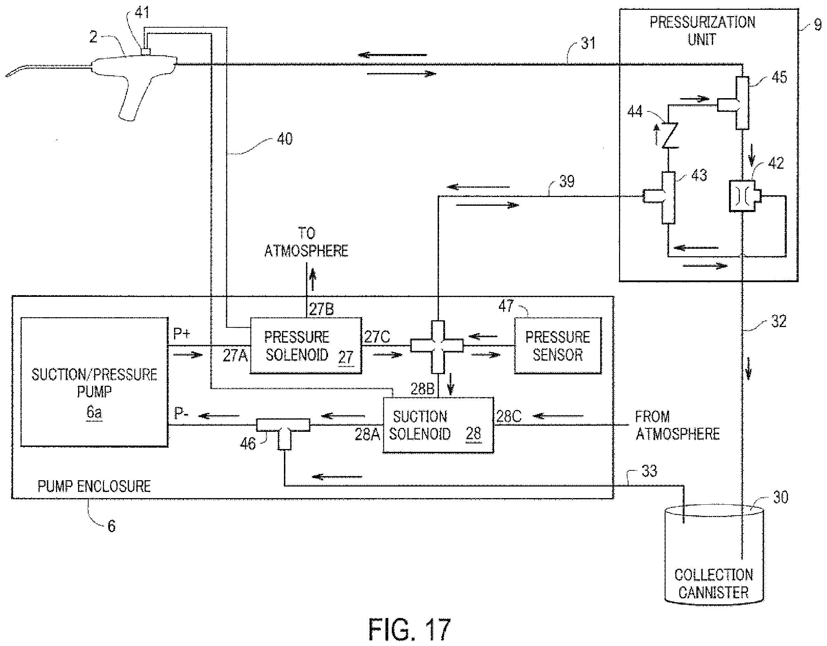

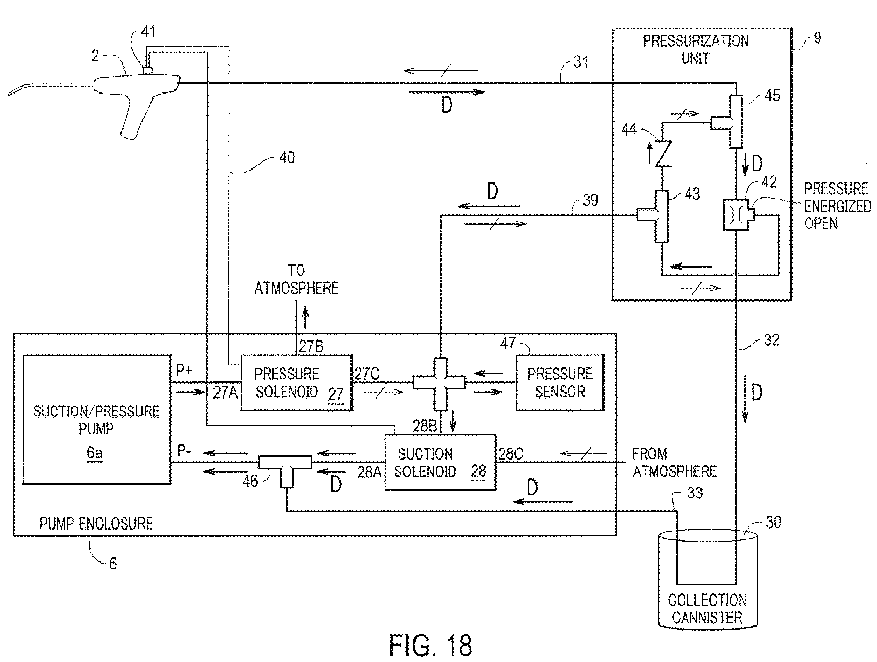

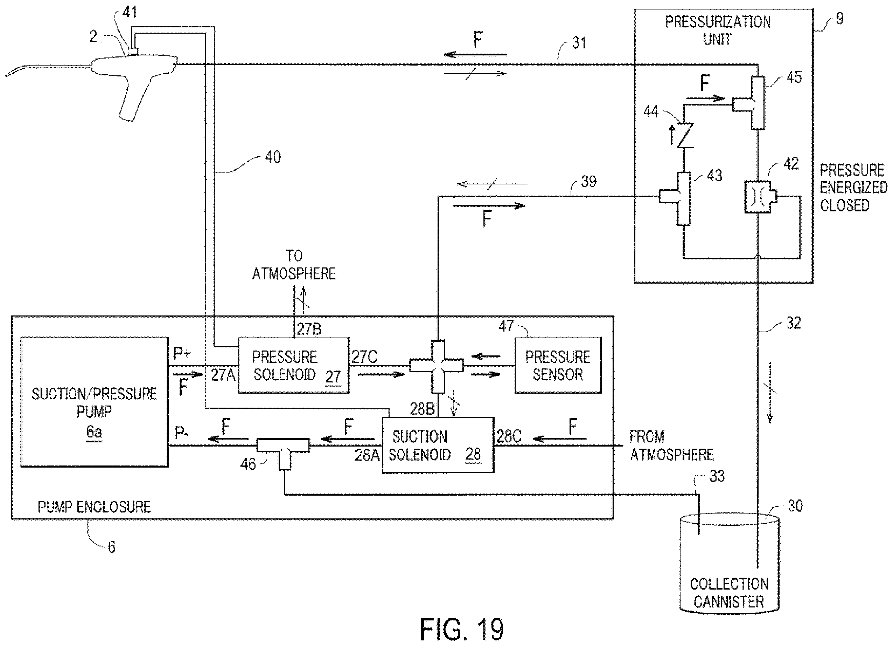

Fig. 17 illustrates an exemplary sixth embodiment, not in accordance with the invention, in which a de-clog button 41 is provided on the handpiece 2 or in the handpiece power cable 5. Different from the previous embodiments, the pressurization unit 9 of the sixth embodiment includes a de-clog valve 42, a first T-junction 43, a one way check valve 44 and a second T-junction 45. The suction source 6 of the sixth embodiment includes a suction/pressure pump 6a, a third T-junction 46, solenoid valves 27 and 28 and a pressure sensor 47. When the system is aspirating tissue normally (without a clog), it typically does so under a normal range of suction pressure (typically around 50% of full vacuum). When the aspiration pathway develops a clog, the pressure will climb to a much higher pressure, as the suction builds and approaches the limits of its vacuum pressure capability. This 'clog pressure' could be automatically sensed by the pressure sensor 47 in the system to initiate a de-clog action without manual intervention. Figs. 18 and 19 respectively illustrate the operation of the sixth embodiment in an aspiration mode (suction providing) and a de-clog mode (pressure providing).

Fig. 18 illustrates the exemplary sixth embodiment in the suction providing or aspiration mode. The arrows D illustrate the flow path of the suction. As seen in Fig. 18, the de-clog valve 42 is open such that suction is provided from the suction/pressure pump 6a through the pressurization unit 9 and to the handpiece 2. In Fig. 18, negative pressure provided through pressure tube 39 causes the de-clog valve 42 to be in an open state. The outlet 27C of solenoid valve 27 is closed and pressurized air exits the solenoid valve 27 to the atmosphere (ATM) through outlet 27B. The inlet 28B of the solenoid valve 28 is open and suction is provided through outlet 28A and inlet 28B of the solenoid valve 28.

Fig. 19 illustrates the exemplary sixth embodiment in a de-clog or pressure providing mode. The arrows F illustrate a flow path of the positive pressure. When the de-clog button 41 is depressed, the solenoid valves 27 and 28 switch from the configurations illustrated in Fig. 18 to the configurations illustrated in Fig. 19. This results in positive pressure being supplied through the pressure tube 39 to the pressurization unit 9. The positive pressure in the pressurization unit 9 causes the de-clog valve 42 to close and causes the positive pressure to be provided through T-junctions 3 and 45 to the first suction tube 31 and to the handpiece 2 to clear a clog. The de-clog valve 42 can include a pressure cuff that surrounds suction tubing. When positive pressure is supplied to the pressure cuff as in Fig. 19, the pressure cuff pinches the suction tubing so that the suction tubing is blocked.

## Claims

1. A surgical instrument comprising:
a hollow tubular member (11) having a cutting blade (14) disposed on a distal end thereof;
a handpiece (2) connected to a proximal end of the hollow tubular member (11) and having a suction passage (15) that connects to the hollow tubular member (11);
a suction pump (6a) that is configured to aspirate tissue that is cut by the cutting blade (14);
a pressurization unit (9) provided between the suction passage (15) and the suction pump (6a), the pressurization unit (9) comprising:
an outer valve portion (17) having a first port (18) that connects to the suction passage (15) and a second port (19) that connects to the suction pump (6a);
a pressurization device (20) connected to the outer valve portion (17), the pressurization device (20) having a movable part (20a) that is configured to generate pressurized fluid when the movable part (20a) is moved;
an inner valve portion (21) that is slidably provided within the outer valve portion (17) and that is configured to move from a first position to a second position, the inner valve portion (21) including:
a first flow passage (23) that extends through the inner valve portion and is configured to connect the first port (18) and the second port (19) of the outer valve portion (17) to each other when the inner valve portion (21) is in the first position; and
a second flow passage (24) that is configured to connect the first port (18) of the outer valve portion (17) to the pressurization device (20) when the inner valve portion (21) is in the second position, the second flow passage (24) extending through the inner valve portion (21) and having a first end that opens at a first end of the inner valve portion and a second end that opens at a side surface of the inner valve portion, wherein
the pressurization unit (9) is configured such that the pressurized fluid from the pressurization device (20) causes the inner valve portion (21) to move from the first position to the second position, thereby providing positive pressure to the hollow tubular member (11).

2. The surgical instrument according to claim 1, wherein
the inner valve portion (21) is biased towards the first position.

3. The surgical instrument according to claim 2, wherein
a spring (22) provides a biasing force to the inner valve portion (21).

4. The surgical instrument according to any one of claims 1-3, wherein
the pressurization device (20) is a compressed air cylinder.

5. The surgical instrument according to any one of claims 1-3, wherein
the pressurization device (20) is a manually compressible syringe (200).

6. The surgical instrument according to any one of claims 1-3, wherein
the pressurization device (20) is a manually compressible inflation bulb.

7. The surgical instrument according to any one of claims 1-6, wherein
the movable part (20a) is manually actuated to cause the inner valve portion (21) to move from the first position to the second position.

8. The surgical instrument according to any one of claims 1-7, wherein the hollow tubular member (11) includes:
a rotatable inner tube (12) having the cutting blade (14) at a distal end thereof; and
an outer tube (13) including a cutting window (16) at a distal end thereof, the rotatable inner tube (12) received within the outer tube (13) so as to align the cutting blade (14) of the rotatable inner tube (12) with the cutting window (16) of the outer tube (13).

9. A method for clearing an obstruction from a surgical instrument, the method comprising:
providing a hollow tubular member (11) having a cutting blade (14) disposed on a distal end thereof;
providing a handpiece (2) connected to a proximal end of the hollow tubular member (11) and having a suction passage (15) that connects to the hollow tubular member (11);
providing a suction pump (6a) that is configured to aspirate tissue that is cut by the cutting blade (14);
providing a pressurization unit (9) between the suction passage (15) and the suction pump (6a), the pressurization unit (9) comprising:
an outer valve portion (17) having a first port (18) that connects to the suction passage (15) and a second port (19) that connects to the suction pump (6a);
a pressurization device (20) connected to the outer valve portion (17), the pressurization device (20) having a movable part (20a) that is configured to generate pressurized fluid when the movable part (20a) is moved;
an inner valve portion (21) that is slidably provided within the outer valve portion (17) and that is configured to move from a first position to a second position, the inner valve portion (21) including:
a first flow passage (23) that extends through the inner valve portion and is configured to connect the first port (18) and the second port (19) of the outer valve portion (17) to each other when the inner valve portion (21) is in the first position; and
a second flow passage (24) that is configured to connect the first port (18) of the outer valve portion (17) to the pressurization device (20) when the inner valve portion (21) is in the second position, the second flow passage (24) extending through the inner valve portion (21) and having a first end that opens at a first end of the inner valve portion and a second end that opens at a side surface of the inner valve portion, and
clearing an obstruction from the surgical instrument by actuating the movable part (20a), which causes the pressurized fluid from the pressurization device (20) to move the inner valve portion (21) from the first position to the second position thereby providing positive pressure to the hollow tubular member (11).

10. The method according to claim 9, further comprising:
manually operating the movable part (20a) to switch the inner valve portion (21) from the first position to the second position.

11. The method according to claim 9 or claim 10, wherein
the pressurization device (20) is a compressed air cylinder.

12. The method according to claim 9 or claim 10, wherein
the pressurization device (20) is a manually compressible syringe (200).

13. The method according to claim 9 or claim 10, wherein
the pressurization device (20) is a manually compressible inflation bulb.

14. The method according to any one of claims 9-13, wherein
the inner valve portion (21) is biased towards the first position.

15. The method according to claim 14, wherein
a spring (22) provides a biasing force to the inner valve portion (21).

## Patentansprüche

1. Chirurgisches Instrument, umfassend:
ein hohles, rohrförmiges Organ (11) mit einer Schneidklinge (14) an seinem distalen Ende,
ein Handstück (2), mit einem proximalen Ende des hohlen, rohrförmigen Organs (11) verbunden und eine Saugpassage (15) aufweisend, die an das hohle, rohrförmige Organ (11) angeschlossen ist,
eine Saugpumpe (6a), dafür eingerichtet, Gewebe abzusaugen, das von der Schneidklinge (14) geschnitten wurde,
eine Überdruckeinheit (9), zwischen der Saugpassage (15) und der Saugpumpe (6a) vorgesehen, wobei die Überdruckeinheit (9) aufweist:
ein äußeres Ventilteil (17) mit einer ersten Öffnung (18), die an die Saugpassage (15) angeschlossen ist, und einer zweiten Öffnung (19), die an die Saugpumpe (6a) angeschlossen ist,
eine Überdruckvorrichtung (20), an das äußere Ventilteil (17) angeschlossen, wobei die Überdruckvorrichtung (20) ein bewegliches Teil (20a) aufweist, das dafür eingerichtet ist, Druckfluid zu erzeugen, wenn das bewegliche Teil (20a) bewegt wird,
ein inneres Ventilteil (21), das innerhalb des äußeren Ventilteiles (17) gleitend vorgesehen ist und das dazu angeordnet ist, sich aus einer ersten Stellung in eine zweite Stellung zu bewegen, wobei das innere Ventilteil (21) aufweist:
eine erste Durchflusspassage (23), die sich durch das innere Ventilteil erstreckt und dafür ausgebildet ist, die erste Öffnung (18) mit der zweiten Öffnung (19) des äußeren Ventilteiles (17) zu verbinden, wenn das innere Ventilteil (21) sich in der ersten Stellung befindet, und
eine zweite Durchflusspassage (24), die dafür ausgebildet ist, die erste Öffnung (18) des äußeren Ventilteiles (17) mit der Überdruckvorrichtung (20) zu verbinden, wenn das innere Ventilteil (21) sich in der zweiten Stellung befindet, wobei sich die zweite Durchflusspassage (24) durch das innere Ventilteil (21) erstreckt und ein erstes Ende aufweist, das an einem ersten Ende des inneren Ventilteiles mündet, und ein zweites Ende, das an einer Seitenfläche des inneren Ventilteiles mündet, wobei
die Überdruckeinheit (9) derart ausgebildet ist, dass das Druckfluid aus der Überdruckvorrichtung (20) das innere Ventilteil (21) dazu veranlasst, sich aus der ersten Stellung in die zweite Stellung zu bewegen, und dadurch das hohle, rohrförmige Organ (11) mit positivem Druck versorgt.

2. Chirurgisches Instrument nach Patentanspruch 1, in dem das innere Ventilteil (21) in die erste Stellung vorgespannt ist.

3. Chirurgisches Instrument nach Patentanspruch 2, in dem eine Feder (22) eine Vorspannkraft auf das innere Ventilteil (21) ausübt.

4. Chirurgisches Instrument nach irgendeinem der Patentansprüche 1 bis 3, in dem die Überdruckvorrichtung (20) ein Druckluftzylinder ist.

5. Chirurgisches Instrument nach irgendeinem der Patentansprüche 1 bis 3, in dem die Überdruckvorrichtung (20) eine manuell zusammendrückbare Spritze (200) ist.

6. Chirurgisches Instrument nach irgendeinem der Patentansprüche 1 bis 3, in dem die Überdruckvorrichtung (20) ein manuell zusammendrückbarer Blaseball ist.

7. Chirurgisches Instrument nach irgendeinem der Patentansprüche 1 bis 6, in dem das bewegliche Teil (20a) manuell betätigt wird, um das innere Ventilteil (21) dazu zu veranlassen, sich aus der ersten Stellung in die zweite Stellung zu bewegen.

8. Chirurgisches Instrument nach irgendeinem der Patentansprüche 1 bis 7, in dem das hohle, rohrförmige Organ (11) umfasst:
ein drehbares Innenrohr (12) mit der Schneidklinge (14) an seinem distalen Ende,
ein Außenrohr (13) mit einem Schneidfenster (16) an seinem distalen Ende, wobei das drehbare Innenrohr (12) im Außenrohr (13) derart aufgenommen wird, dass die Schneidklinge (14) des drehbaren Innenrohres (12) mit dem Schneidfenster (16) des Außenrohres (13) ausgerichtet ist.

9. Verfahren zum Beseitigen einer Verstopfung eines chirurgischen Instrumentes, wobei das Verfahren umfasst:
Bereitstellen eines hohlen, rohrförmigen Organs (11) mit einer Schneidklinge (14) an seinem distalen Ende,
Bereitstellen eines Handstückes (2), mit einem proximalen Ende des hohlen, rohrförmigen Organs (11) verbunden und eine Saugpassage (15) aufweisend, die an das hohle, rohrförmige Organ (11) angeschlossen ist,
Bereitstellen einer Saugpumpe (6a), dafür eingerichtet, Gewebe abzusaugen, das von der Schneidklinge (14) geschnitten wurde,
Bereitstellen einer Überdruckeinheit (9) zwischen der Saugpassage (15) und der Saugpumpe (6a), wobei die Überdruckeinheit (9) aufweist:
ein äußeres Ventilteil (17) mit einer ersten Öffnung (18), die an die Saugpassage (15) angeschlossen ist, und einer zweiten Öffnung (19), die an die Saugpumpe (6a) angeschlossen ist,
eine Überdruckvorrichtung (20), an das äußere Ventilteil (17) angeschlossen, wobei die Überdruckvorrichtung (20) ein bewegliches Teil (20a) aufweist, das dafür eingerichtet ist, Druckfluid zu erzeugen, wenn das bewegliche Teil (20a) bewegt wird,
ein inneres Ventilteil (21), das innerhalb des äußeren Ventilteiles (17) gleitend vorgesehen ist und das dazu angeordnet ist, sich aus einer ersten Stellung in eine zweite Stellung zu bewegen,
wobei das innere Ventilteil (21) aufweist:
eine erste Durchflusspassage (23), die sich durch das innere Ventilteil erstreckt und dafür ausgebildet ist, die erste Öffnung (18) mit der zweiten Öffnung (19) des äußeren Ventilteiles (17) zu verbinden, wenn das innere Ventilteil (21) sich in der ersten Stellung befindet, und
eine zweite Durchflusspassage (24), die dafür ausgebildet ist, die erste Öffnung (18) des äußeren Ventilteiles (17) mit der Überdruckvorrichtung (20) zu verbinden, wenn das innere Ventilteil (21) sich in der zweiten Stellung befindet, wobei sich die zweite Durchflusspassage (24) durch das innere Ventilteil (21) erstreckt und ein erstes Ende aufweist, das an einem ersten Ende des inneren Ventilteiles mündet, und ein zweites Ende, das an einer Seitenfläche des inneren Ventilteiles mündet, und
Beseitigen einer Verstopfung aus dem chirurgischen Instrument durch Betätigung des beweglichen Teils (20a), was das Druckfluid aus der Überdruckvorrichtung (20) dazu veranlasst, das innere Ventilteil (21) aus der ersten Stellung in die zweite Stellung zu bewegen und dadurch das hohle, rohrförmige Organ (11) mit positivem Druck zu versorgen.

10. Verfahren nach Patentanspruch 9, außerdem umfassend:
manuelle Betätigung des beweglichen Teils (20a) zum Schieben des inneren Ventilteiles (21) aus der ersten Stellung in die zweite Stellung.

11. Verfahren nach Patentanspruch 9 oder 10, in dem die Überdruckvorrichtung (20) ein Druckluftzylinder ist.

12. Verfahren nach Patentanspruch 9 oder 10, in dem die Überdruckvorrichtung (20) eine manuell zusammendrückbare Spritze (200) ist.

13. Verfahren nach Patentanspruch 9 oder 10, in dem die Überdruckvorrichtung (20) ein manuell zusammendrückbarer Blaseball ist.

14. Verfahren nach irgendeinem der Patentansprüche 9 bis 13, in dem das innere Ventilteil (21) in die erste Stellung vorgespannt ist.

15. Verfahren nach Patentanspruch 14, in dem eine Feder (22) eine Vorspannkraft auf das innere Ventilteil (21) ausübt.

## Revendications

1. Instrument chirurgical comprenant :
un élément tubulaire creux (11) ayant une lame de coupe (14) disposée sur une extrémité distale de celui-ci ;
une pièce à main (2) reliée à une extrémité proximale de l'élément tubulaire creux (11) et ayant un passage d'aspiration (15) qui est relié à l'élément tubulaire creux (11) ;
une pompe d'aspiration (6a) qui est configurée pour aspirer du tissu qui est coupé par la lame de coupe (14) ;
une unité de mise sous pression (9) prévue entre le passage d'aspiration (15) et la pompe d'aspiration (6a), l'unité de mise sous pression (9) comprenant :
une portion de valve extérieure (17) ayant un premier orifice (18) qui est relié au passage d'aspiration (15) et un deuxième orifice (19) qui est relié à la pompe d'aspiration (6a) ;
un dispositif de mise sous pression (20) relié à la portion de valve extérieure (17), le dispositif de mise sous pression (20) ayant une partie mobile (20a) qui est configurée pour générer un fluide sous pression lorsque la partie mobile (20a) est déplacée ;
une portion de valve intérieure (21) qui est prévue de manière coulissante à l'intérieur de la portion de valve extérieure (17) et qui est configurée pour se déplacer d'une première position à une deuxième position, la portion de valve intérieure (21) comprenant :
un premier passage de flux (23) qui s'étend à travers la portion de valve intérieure et est configuré pour relier le premier orifice (18) et le deuxième orifice (19) de la portion de valve extérieure (17) l'un à l'autre lorsque la portion de valve intérieure (21) est dans la première position ; et
un deuxième passage de flux (24) qui est configuré pour relier le premier orifice (18) de la portion de valve extérieure (17) au dispositif de mise sous pression (20) lorsque la portion de valve intérieure (21) est dans la deuxième position, le deuxième passage de flux (24) s'étendant à travers la portion de valve intérieure (21) et ayant une première extrémité qui s'ouvre à une première extrémité de la portion de valve intérieure et une deuxième extrémité qui s'ouvre à une surface latérale de la portion de valve intérieure, dans lequel
l'unité de mise sous pression (9) est configurée de sorte que le fluide sous pression du dispositif de mise sous pression (20) amène la portion de valve intérieure (21) à se déplacer de la première position à la deuxième position, fournissant ce faisant une pression positive à l'élément tubulaire creux (11).

2. Instrument chirurgical selon la revendication 1, dans lequel la portion de valve intérieure (21) est sollicitée vers la première position.

3. Instrument chirurgical selon la revendication 2, dans lequel un ressort (22) fournit une force de sollicitation à la portion de valve intérieure (21).

4. Instrument chirurgical selon l'une quelconque des revendications 1-3, dans lequel le dispositif de mise sous pression (20) est un cylindre à air comprimé.

5. Instrument chirurgical selon l'une quelconque des revendications 1-3, dans lequel le dispositif de mise sous pression (20) est une seringue compressible manuellement (200).

6. Instrument chirurgical selon l'une quelconque des revendications 1-3, dans lequel le dispositif de mise sous pression (20) est une ampoule de gonflage compressible manuellement.

7. Instrument chirurgical selon l'une quelconque des revendications 1-6, dans lequel la partie mobile (20a) est actionnée manuellement pour amener la portion de valve intérieure (21) à se déplacer de la première position à la deuxième position.

8. Instrument chirurgical selon l'une quelconque des revendications 1-7, dans lequel l'élément tubulaire creux (11) comprend :
un tube intérieur rotatif (12) ayant une lame de coupe (14) à une extrémité distale de celui-ci ; et
un tube extérieur (13) comprenant une fenêtre de coupe (16) à une extrémité distale de celui-ci, le tube intérieur rotatif (12) étant reçu à l'intérieur du tube extérieur (13) de façon à aligner la lame de coupe (14) du tube intérieur rotatif (12) sur la fenêtre de coupe (16) du tube extérieur (13).

9. Procédé de dégagement d'une obstruction d'un instrument chirurgical, le procédé comprenant :
la fourniture d'un élément tubulaire creux (11) ayant une lame de coupe (14) disposée sur une extrémité distale de celui-ci ;
la fourniture d'une pièce à main (2) reliée à une extrémité proximale de l'élément tubulaire creux (11) et ayant un passage d'aspiration (15) qui est relié à l'élément tubulaire creux (11) ;
la fourniture d'une pompe d'aspiration (6a) qui est configurée pour aspirer du tissu qui est coupé par la lame de coupe (14) ;
la fourniture d'une unité de mise sous pression (9) entre le passage d'aspiration (15) et la pompe d'aspiration (6a), l'unité de mise sous pression (9) comprenant :
une portion de valve extérieure (17) ayant un premier orifice (18) qui est relié au passage d'aspiration (15) et un deuxième orifice (19) qui est relié à la pompe d'aspiration (6a) ;
un dispositif de mise sous pression (20) relié à la portion de valve extérieure (17), le dispositif de mise sous pression (20) ayant une partie mobile (20a) qui est configurée pour générer un fluide sous pression lorsque la partie mobile (20a) est déplacée ;
une portion de valve intérieure (21) qui est prévue de manière coulissante à l'intérieur de la portion de valve extérieure (17) et qui est configurée pour se déplacer d'une première position à une deuxième position, la portion de valve intérieure (21) comprenant :
un premier passage de flux (23) qui s'étend à travers la portion de valve intérieure et est configuré pour relier le premier orifice (18) et le deuxième orifice (19) de la portion de valve extérieure (17) l'un à l'autre lorsque la portion de valve intérieure (21) est dans la première position ; et
un deuxième passage de flux (24) qui est configuré pour relier le premier orifice (18) de la portion de valve extérieure (17) au dispositif de mise sous pression (20) lorsque la portion de valve intérieure (21) est dans la deuxième position, le deuxième passage de flux (24) s'étendant à travers la portion de valve intérieure (21) et ayant une première extrémité qui s'ouvre à une première extrémité de la portion de valve intérieure et une deuxième extrémité qui s'ouvre à une surface latérale de la portion de valve intérieure, et
le dégagement d'une obstruction de l'instrument chirurgical par actionnement de la partie mobile (20a) qui amène le fluide sous pression du dispositif de mise sous pression (20) à déplacer la portion de valve intérieure (21) de la première position à la deuxième position fournissant ce faisant une pression positive à l'élément tubulaire creux (11).

10. Procédé selon la revendication 9, comprenant en outre :
l'actionnement manuel de la partie mobile (20a) à commuter la portion de valve intérieure (21) de la première position à la deuxième position.

11. Procédé selon la revendication 9 ou 10, dans lequel
le dispositif de mise sous pression (20) est un cylindre à air comprimé.

12. Procédé selon la revendication 9 ou 10, dans lequel
le dispositif de mise sous pression (20) est une seringue compressible manuellement (200).

13. Procédé selon la revendication 9 ou 10, dans lequel
le dispositif de mise sous pression (20) est une ampoule de gonflage compressible manuellement.

14. Procédé chirurgical selon l'une quelconque des revendications 9-13, dans lequel
la portion de valve intérieure (21) est sollicitée vers la première position.

15. Procédé selon la revendication 14, dans lequel
un ressort (22) fournit une force de sollicitation à la portion de valve intérieure (21).
